# EUROPÄISCHE PATENTSCHRIFT

(11) **EP 0 630 627 B1**
(45) Veröffentlichungstag und Bekanntmachung des Hinweises auf die Patenterteilung: **07.01.1999**
(21) Anmeldenummer: 94102364.0
(22) Anmeldetag: 17.02.1994
(51) Int. Cl.: A61F 9/06, G02F 1/00

(54) **Electro-optische Blendschutzvorrichtung für Schweiss- und Schneidbrennertechnik**
Electro optical glare protection for welding
Ecran électro optique de protection pour soudage

(30) Priorität: 14.05.1993 CH 1488/93
(43) Veröffentlichungstag der Anmeldung: 28.12.1994
(73) Patentinhaber: XELUX HOLDING AG, 6300 Zug (CH)
(72) Erfinder: Gunz, Stefan, CH-8820 Wädenswil (CH); Ghisleni, Livio, CH-8832 Wilen b. Wollerau (CH); Castelberg, Manfred, CH-8820 Wädenswil (CH)
(74) Vertreter: Ritscher, Thomas, Dr.

(56) Entgegenhaltungen:
- EP-A- 0 157 744
- DE-A- 2 747 614
- US-A- 4 237 557

## Beschreibung

Die vorliegende Erfindung betrifft eine Blendschutzvorrichtung gemäss Oberbegriff des Anspruchs 1.

Blendschutzvorrichtungen werden bevorzugt in der Schweiss- und Schneidbrennertechnik eingesetzt, finden aber auch in der Militär- und in Unterhaltungsindustrien ihre Verwendung. In der Regel wird bei diesen Blendschutzvorrichtungen die Strahlung oberhalb 780 nm (IR) und unterhalb 365 nm (UV) weggefiltert und nur die Strahlung im sichtbaren Bereich abgeblendet.

Bekannt und bspw. in der DE-2'606'416 beschrieben sind insbesondere Blendschutzvorrichtungen, mit einer Flüssigkristallzelle, welche zwischen zwei polarisierenden Folien angeordnet ist. Dieser Flüssigkristall weist eine frequenzabhängige Anisotropie der dielektrischen Konstanten auf, d.h. ändert seine homöotrope Orientierung in Abhängigkeit der angelegten Frequenz des elektrischen Wechselfeldes. Die derart realisierbaren Umschaltzeiten, d.h. Abdunklungszeit resp. Aufhellzeit betragen etwa 50 msec.

In der US-3,575,491 wird eine elektronische Schaltung zum Betrieb von Flüssigkristallzellen beschrieben, um die Umschaltzeiten auf bis zu 0,5 msec zu reduzieren. Diese Schaltung arbeitet mit einer Spannung von über 125 V.

In allen diesen Blendschutzvorrichtungen ist die Umschaltzeit gerätespezifisch, d.h. hängt von der jeweiligen Konstruktion, elektronischen Schaltung und den verwendeten Bauteilen ab. Die heute gebräuchlichen Blendschutzvorrichtungen zeichen sich durch ausserordentlich kurze Umschaltzeiten und niedere Betriebsleistungen aus.

Es zeigt sich aber, dass die kurzen Umschaltzeiten wohl für die Abdunklungsphase absolut erforderlich sind, für die Aufhellphase aber nicht immer erwünscht sind. Insbesondere führen kurze Aufhellzeiten beim Schweissen von hellem Schweissgut mit langer Nachglühzeit unmittelbar nach Beendigung des Schweissvorgangs zu intensiven Blendungen.

Anderseits können aber gerade kurze Aufhellzeiten, bspw. beim Punkt- resp. Heftschweissen, erforderlich sein, um ein rasches Arbeiten mit kurzen Taktzeiten überhaupt zu ermöglichen.

Es ist deshalb auch schon vorgeschlagen worden, bspw. in der US 4,237,557, eine Blendschutzvorrichtung zu schaffen, bei welcher das Aufhellen der verdunkelten Flüssigkristallzelle um zwei bis drei Sekunden verzögert ist. Auch bei dieser Anordnung wird die Aufhellzeit durch die elektronische Schaltung fest vorgegeben.

Deingegenüber Aufgabe der vorliegenden Erfindung eine Vorrichtung zu schaffen, welche automatisch erkennen kann, wie lange die Aufhellzeit in konkretem Fall sein sollte, um den Benutzer der Blendschutzvorrichtung sicher zu schützen, jedoch nicht in seinem Arbeitsrhythmus zu behindern.

Diese Aufgabe wird erfindungsgemäss mit einer Vorrichtung gelöst, welche die kennzeichnenden Merkmale des Anspruchs 1 aufweist und insbesondere die absolute Lichtintensität, resp. Lichtmenge und die Schweissdauer erfasst und diese Parameter mittels einer geeigneten Elektronik logisch und/oder zeitlich sinnvoll miteinander verknüpft, um damit die Aufhellzeit den gegebenen Umständen optimal anzupassen.

In einer weiterführenden Ausführungsform wird auch die Veränderung der Lichtintensität im Verlauf des Schweissvorgangs und die Länge des Schweissunterbruchs erfasst und ausgewertet.

Die erfindungsgemässe Vorrichtung erlaubt damit auch die unterschiedliche Helligkeit des Schweissbogens (verursacht durch unterschiedlich starke Ströme, verschiedene Materialien, etc.) für die Steuerung der Aufhellzeit zu berücksichtigen und schafft damit eine universell einsetzbare Blendschutzvorrichtung.

Insbesondere ermöglicht die erfindungsgemässe Blendschutzvorrichtung ein behinderungsfreies Arbeiten und gewährleistet gleichzeitig die erforderliche Sicherheit beim professionellen Einsatz dieser Vorrichtungen.

Die erfindungsgemässe Blendschutzvorrichtung soll im folgenden anhand der Figuren und einem Ausführungsbeispiel näher erläutert werden. Dabei zeigt:
Figur 1: ein Blockschaltbild einer Blendschutzvorrichtung bekannter Art;
Figur 2: ein Blockschaltbild einer erfindungsgemässen Blendschutzvorrichtung.
Figur 3: ein Blockschaltbild einer geeigneten Kontrolleinheit.

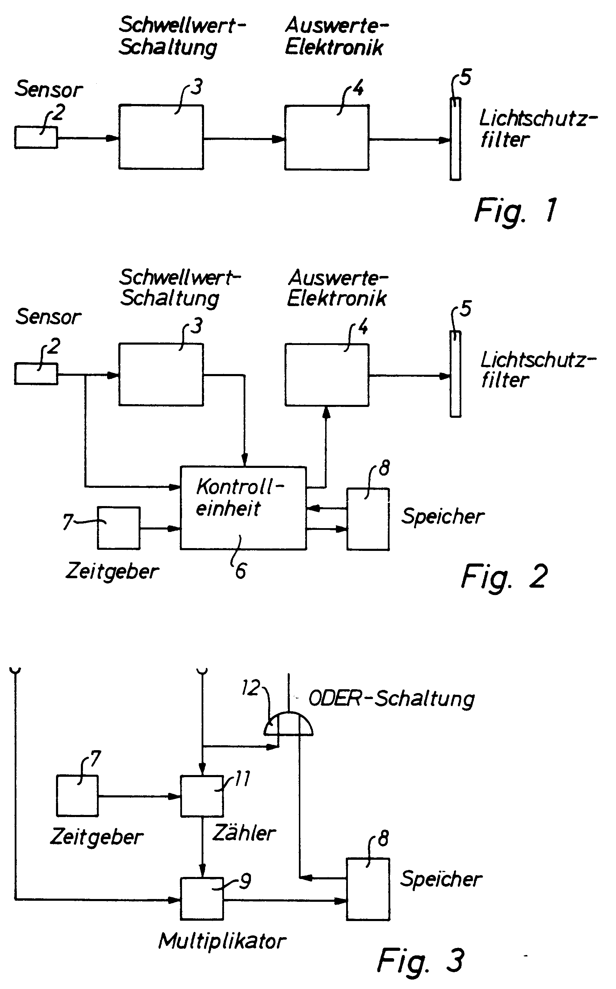

Die in Figur 1 prinzipiell dargestellte Blendschutzvorrichtung bekannter Art weist einen Sensor 2 auf, welcher die im Blickfeld des Benutzers erzeugte Lichtmenge detektiert und ein entsprechendes Signal erzeugt. Dieses Signal wird einer Detektor-Elektronik 3 zugeführt, welche im wesentlichen eine Schwellwertschaltung umfasst. Das von dieser Schwellwertschaltung erzeugte Ein/Aus-Signal wird einer Auswerte-Elektronik 4 zugeführt, welche dieses Ein/Aus-Signal derart aufbereitet, dass damit ein Lichtschutzfilter 5 möglichst rasch geschaltet werden kann.

Demgegenüber weist die in Figur 2 dargestellte Blendschutzvorrichtung eine Kontrolleinheit 6 auf, welche mit einem Zeitgeber 7 und Speicher 8 verbunden ist. Diese Kontrolleinheit 6 erfasst das vom Sensor 2 erzeugte Signal direkt und wertet dieses weiter aus. Insbesondere ermittelt diese Kontrolleinheit 6 die Intensität des auf den Sensor 2 auftreffenden Lichts und generiert ein dieser Lichtmenge entsprechendes Signal, dessen Wert im Speicher 8 abgelegt wird, um - in vorbestimmten Grenzen - die Aufhellzeit des Lichtschutzfilters 5 entsprechend zu verlängern.

Das von der Detektor-Elektronik 3 erzeugte Signal wird ebenfalls der Kontrolleinheit 6 zugeführt und in für den Fachmann naheliegender Weise mit dem Signal des Sensors 2 logisch und/oder zeitlich verknüpft, um die Aufhellzeit in gewünschter und/oder erforderlicher Weise zu steuern.

In einer weiterführenden Ausführungsform werden die erfassten und/oder erzeugten Signale verwendet, um den Dunkel/Hell-Übergang während der Aufhellzeit zu steuern, d.h. einen sprungartigen resp. schleifenden Uebergang zu erzeugen.

Insbesondere kann dazu die detektierte Dauer des Schweissvorgangs und/oder der Verlauf des vom Sensor 2 erzeugten Signals zu Beginn, während und beim Beenden der Blendphase erfasst und der Erzeugung eines Reset-Signals dienen.

In einer vereinfachten Ausführungsform ist die Steilheit des Dunkel/Hell-Uebergangs direkt von der generierten Aufhellzeit abhängig. Es versteht sich auch, dass bei Blendschutzvorrichtungen, wie sie von Schweissern professionell verwendet werden, eine manuelle Schutzstufeneinstellung vorgesehen ist. Erfindungsgemäss kann die vom Anwender gewählte Schutzstufe und deren manuelle Feinregulierung zusätzlich für die Steuerung der Steilheit des Dunkel/Hell-Uebergangs verwendet werden.

Figur 3 zeigt eine Ausführungsform für die zeitliche und/oder logische Verknüpfung der ersten und zweiten Schaltsignale. In dieser Ausführungsform wird das von der Detektorschaltung 3 erzeugte zweite Schaltsignal einer logischen ODER-Schaltung 12 zugeleitet und erlaubt damit das unmittelbare Verdunkeln des Lichtschutzfilters 5 im Falle einer Blendsituation. Das Ein/Aus-Signal der Detektor-Elektronik 3 wird auch einem mit dem Zeitgeber 7 verbundenen Zeitzähler 11 zugeleitet. Dieser Zeitzähler ist wiederum mit einer Multiplikatorschaltung 9 verbunden und aktiviert diese während dieser Blendsituation, im folgenden Blendphase genannt. Gleichzeitig wird die Multiplikatorschaltung 9 mit dem vom Sensor 2 generierten ersten Schaltsignal direkt gespeist und erzeugt einen entsprechenden Signalwert, welcher im Speicher 8 abgelegt wird.

Generiert die Detektor-Elektronik 3 ein Aus-Signal, wird über die ODER-Schaltung 12 ein Aufhellen des Lichtschutzfilters 5 solange verhindert, bis ein dem Speicherinhalt entsprechend verzögertes RESET-Signal den Zähler 11 wieder auf Null setzt und damit die Multiplikatorschaltung 9 desaktiviert. Die Verzögerung des RESET-Signals entspricht im vorliegenden Beispiel dem Produkt aus Zeit der Blendphase und Intensität des Blendlichts.

Die elektronische Verdrahtung der Kontrolleinheit 6 liegt im Bereich des fachmännischen Könnens und kann sowohl aus Einzelbausteinen, integrierten Teilbausteinen oder einem geeigneten Mikroprozessor aufgebaut sein. Die Verwendung weiterer Sensoren 2 oder Speichereinheiten 8 zur Weiterentwicklung und Optimierung der erfindungsgemässen Blendschutzvorrichtung ist für den Fachmann naheliegend.

Die erfindungsgemässe Blendschutzvorrichtung ist damit universell einsetzbar, d.h. erlaubt beim Punkt- und Heftschweissen einen raschen und ungehinderten Arbeitsrhythmus und stellt die Verdunkelung während der augengefährdenden Nachglühzeit beim Langzeitschweissen sicher.

## Patentansprüche

1. Blendschutzvorrichtung geeignet für Brillen, Helme und Masken, wie sie in der Schweiss- und Schneidbrennertechnik verwendet werden, mit einem Photosensor (2), mit einer Detektor-Elektronik (3) zur Erzeugung eines Abblendsignals, mit einer Auswerte-Elektronik (4) zur Steuerung eines elektrooptischen Lichtschutzfilters (5), wobei zur Steuerung der Aufhellzeit des Lichtschutzfilters (5) eine Kontrollvorrichtung (6) vorgesehen ist, dadurch gekennzeichnet, dass die Kontrollvorrichtung (6) mindestens die Intensität des auf den Sensor (2) auftreffenden Lichtes erfasst und mit einem Zeitgeber (7) verbunden ist, um mindestens die Dauer des von der Detektor-Elektronik (3) erzeugten Abblendsignals zu erfassen, und welche Kontrollvorrichtung (6) eine Multiplikatorschaltung (9) und einen Speicher (8) umfasst, um die erfasste Intensität das auf den Sensor (2) auftreffenden Lichtes und die erfasste Dauer des von der Detektor-Elektronik (3) erzeugten Abblendsignals logisch und/oder zeitlich miteinander zu verknüpfen.

2. Blendschutzvorrichtung nach Anspruch 1, dadurch gekennzeichnet, dass die Kontrollvorrichtung (6) den Verlauf der auf den Sensor (2) auftreffenden Lichtintensität erfasst und ein Signal für die Steuerung der Aufhellzeit des Lichtschutzfilters (5) erzeugt.

3. Blendschutzvorrichtung nach Anspruch 1, dadurch gekennzeichnet, dass die Kontrollvorrichtung (6) nach jeder Blendphase ein Reset-Signal erzeugt, welches die Multiplikatorschaltung (9) desaktiviert, im den Speicher (8) zurückzusetzen.

4. Blendschutzvorrichtung nach Anspruch 3, dadurch gekennzeichnet, dass das Reset-Signal gegenüber dem Blendphasenende eine Verzögerung aufweist.

5. Blendschutzvorrichtung nach Anspruch 2, dadurch gekennzeichnet, dass die Kontrollvorrichtung (6) den absoluten Wert der auf den Sensor (2) auftreffenden Lichtmenge erfasst und ein Signal für die Steuerung der Aufhellzeit des Lichtschutzfilters (5) erzeugt.

6. Blendschutzvorrichtung nach einem der Ansprüche 1 bis 5, dadurch gekennzeichnet, dass die Kontrollvorrichtung (6) ein Signal generiert, welches die Steilheit des Dunkel/Hell-Übergangs während der Aufhellzeit steuert.

7. Blendschutzvorrichtung nach Anspruch 6, dadurch gekennzeichnet, dass dieses Signal von einer manuelleinstellbaren Schutzstufeneinstellung abhängig ist.

## Claims

1. Glare protection device suitable for glasses, helmets and masks as are used in the welding and cutting torch technology, with a photosensor (2), with detector electronics (3) for generating a darkening signal, with evaluation electronics (4) for controlling an electro-optical light protective filter (5), whereby for controlling the brightening time of the light protective filter (5) a control device (6) is provided, characterized in that the control device (6) registers at least the intensity of the light impacting on the sensor (2) and which control device is connected to a timer (7), in order to at least detect the duration of the darkening signal generated by the detector electronics (3), and which control device (6) comprises a multiplicator circuit (9) and a memory (8) in order to logically and/or chronologically combine the registered intensity of the light impacting the sensor (2) and the registered duration of the darkening signal generated by the detection electronics (3).

2. Glare protection device according to claim 1, characterized in that the control device (6) registers the progression of the light intensity impacting on the sensor (2) and generates a signal for controlling the brightening time of the light protective filter (5).

3. Glare protection device according to claim 1, characterized in that the control device (6) generates a reset signal after each glare phase which deactivates the multiplicator circuit (9), thus resetting the memory (8).

4. Glare protection device according to claim 3, characterized in that the reset signal is delayed with respect to the end of the glare phase.

5. Glare protection device according to claim 2, characterized in that the control device (6) registers the absolute value of the amount of light impacting on the sensor (2) and generates a signal for controlling the bright-up time of the light protection filter (5).

6. Glare protection device according to one of claims 1 to 5, characterized in that the control device (6) generates a signal which controls the steepness of the dark / bright transition during the bright-up time.

7. Glare protection device according to claim 6, characterized in that this signal is dependent upon a manually adjustable setting of the protection factor.

## Revendications

1. Dispositif de protection contre l'éblouissement adapté pour lunettes, casques et masques tels qu'ils sont employés dans les techniques de soudage et d'oxycoupage, comprenant un capteur optique (2), un circuit électronique de détection (3) qui génère un signal anti-éblouissement, un circuit électronique d'évaluation (4) pour le contrôle du filtre de protection électro-optique (5), un dispositif de contrôle (6) étant prévue pour contrôler la durée d'éclaircissement du filtre optique de protection (5), caractérisé en ce que le dispositif de contrôle (6) détecte au moins l'intensité de la lumière tombant sur le capteur (2) et est relié à une horloge (7), afin de détecter au moins la durée du signal d'anti-éblouissement créée par le circuit électronique de détection (3), et que ledit dispositif de contrôle (6) comprend un circuit multiplicateur (9) et une mémoire (8) afin de combiner logiquement et/ou dans le temps l'intensité détectée de la lumière tombant sur le capteur (2) et la durée détectée du signal d'anti-éblouissement créé par le circuit électronique de détection (3).

2. Dispositif de protection contre l'éblouissement selon la revendication 1, caractérisé en ce que le dispositif de contrôle (6) détecte la variation dans le temps de l'intensité lumineuse tombant sur le capteur (2) et crée un signal pour le contrôle de la durée d'éclaircissement du filtre optique de protection (5).

3. Dispositif de protection contre l'éblouissement selon la revendication 1, caractérisé en ce que le dispositif de contrôle (6) génère après chaque phase d'éblouissement un signal de reset qui désactive le circuit multiplicateur (9) afin de remettre la mémoire (8).

4. Dispositif de protection contre l'éblouissement selon la revendication 3, caractérisé en ce que le signal de reset présente un retard par rapport à la fin de la phase d'éblouissement.

5. Dispositif de protection contre l'éblouissement selon la revendication 2, caractérisé en ce que le dispositif de contrôle (6) détecte la valeur totale de la quantité de lumière tombant sur le capteur (2) et génère un signal de contrôle de la durée d'éclaircissement du filtre optique de protection (5).

6. Dispositif de protection contre l'éblouissement selon l'une des revendications 1 à 5, caractérisé en ce que le dispositif de contrôle (6) génère un signal qui contrôle la vitesse de la variation claire/foncée lors de la durée d'éclaircissement.

7. Dispositif de protection contre l'éblouissement selon la revendication 6, caractérisé en ce que ce signal est dépendant d'un réglage, capable d'être effectué manuellement, du degré de protection.
